# EUROPEAN PATENT SPECIFICATION

(11) **EP 2 471 453 B1**
(45) Date of publication and mention of the grant of the patent: **15.01.2014**
(21) Application number: 11154815.2
(22) Date of filing: 17.02.2011
(51) Int. Cl.: A61B 5/053, A61N 1/30, A61B 5/11

(54) **Apparatus for skin care equipped with the measuring function of skin conductivity and method of operating thereof**
Vorrichtung zur Hautpflege mit einer Messfunktion der Hautleitfähigkeit und Betriebsverfahren dafür
Appareil pour soins cutanés équipé de la fonction de mesure de la conductivité de la peau et son procédé de fonctionnement

(30) Priority: 28.12.2010 KR 20100136985
(43) Date of publication of application: 04.07.2012
(73) Proprietor: Lotts Co., Ltd., Yeoksam-dong Kangnam-gu Seoul 135-513 (KR)
(72) Inventor: Hwang,, Seung Young, 135-513, SEOUL (KR)
(74) Representative: Kransell & Wennborg KB

(56) References cited:
- WO-A1-00/30711
- WO-A1-2004/110205
- KR-A- 20040 108 505
- US-A- 4 141 359
- US-A1- 2008 058 699

## Description

### BACKGROUND OF THE INVENTION

### 1. The Technical Field

The present invention is related to a skin care apparatus to measure a level of the skin conductivity (galvanic skin response) and its operational instruction. The apparatus allows a user to measure the skin conductivity to anticipate the level of the user's skin moisture and the deposited ions for applying the suitable voltage and reacting time for the best skin treatment.

### 2. Description of the Related Prior Art.

The conventional skin care devises, typically portable galvanic skin care devises, would be induced to penetrate the galvanic ions into a users' skin for easily absorbing the cosmetic materials. This kind of devise is used to perform the skin treatments depending on a naked eye inspection or touch-feeling experience without exactly understanding the user's current conditions of the skin moisture and/or depositions of the ions.

Therefore, the inspection for skin treatment varies depending on a user's skin condition or the operator's experience. With the inaccurate inspections, there are certainly many problems incident that the skin treatment would be often unnecessarily excessive treating, or contrary the skin treatment would be insufficient treating.

The present invention precisely inspects the user's conditions of the skin moisture and the accumulated ions prior to or during performing the skin treatment. Thus, the operator is able to select an optimum applying voltage and time for the best mode of ion deposition. Further, the user can measure the skin conductivity, periodically with constant intervals, during the skin treatment, for adjusting the amount of the ion supply, the applying voltage and times.

Document US 4 141 359 discloses an iontophoresis device for topical administration of ionic drugs or chemicals through epidermal tissue for anesthetizing or sterilizing local tissue or for applying various medicaments without mechanical penetration.

Document US 2008/058699 discloses an iontophoresis apparatus and method that is suited to deliver a plurality of treatment methods such as body site conductivity enhancement followed by low voltage iontophoresis.

Document WO 2004/110205 discloses a skin-care device having an ion penetration function to penetrate an ionized substance underneath skin.

Document WO 00/30711 discloses a handheld skin treatment system and method.

Document KR 2004 0108505 discloses a skin care apparatus having ion-transmission function to minimize unnecessary electric power consumption and to detect whether the apparatus is used or not by user.

### SUMMARY OF THE INVENTION

Hereinafter, in order to solve the aforementioned problems, the objective of the present invention is providing an apparatus for skin care having a measuring function of the skin conductivity.

According to the present invention an apparatus according to the appended claims is provided.

It is another objective of the present invention to provide an apparatus for skin care further provided: an ultrasonic unit (50), which is controlled by the microcontroller.

It is further another objective of the present invention to provide an apparatus for skin care further provided: a laser generating unit (153), which is controlled by the microcontroller, for emitting a laser beam on the skin.

It is further another objective of the present invention to provide an apparatus for skin care further provided: a low/high frequency pulse generating unit (152) for generating electrical oscillations of the low or high frequency to stimulate the user's skin, said low/high frequency pulse generating unit (152) being controlled by the microcontroller, wherein an electrical circuit (Q1) of the galvanic unit (60) will be shut down to disconnect the hand electrode (J7), when the low/high frequency pulse generating unit (152) operates.

It is further another objective of the present invention to provide an apparatus for skin care further provided: a skin contacting element for the ions deposition and the ultrasonic vibration (diaphragm and contacting electrode) made of a single piece, in order to accomplish the functions of the ions deposition and the ultrasonic vibration, simultaneously.

It is further another objective of the present invention to provide an apparatus for skin care, wherein the skin conductivity measuring unit (70) detects the current between the hand electrode (J7) and the skin electrode (J6) of the galvanic unit (60), amplifies the detected current by the amplifier (71, 72) and inputs the amplified current to the control means (2).

In order to solve the aforementioned problems, the further another objective of the present invention is providing a method for skin care having a measuring function of the skin conductivity. According to the present invention a method according to the appended claims is provided.

### THE EFFECT OF THE INVENTION:

As described in detail, the skin care equipment of the present invention has a merit that it can accurately measure the user's skin moisture level by simply contacting the electrode on the user's skin. Therefore, it is possible to select the optimal ion induce voltage and a suitable ion reacting time for the best skin treatment.

In addition, the skin care equipment of the present invention has further included several functions of the ion laser emitting capability, the ultrasonic vibrator, the low/high frequency electrical vibration, at the same time for the maximum effect.

For reference, the galvanic ion induction supplies the nutrition to the user's skin, while the galvanic ions are inducing. Conversely, the impurities are removing from the skin organs. The ultrasonic wave may be used for massaging orsoothing. The lowfrequency wave has effects to anti-wrinkle, obesity, improve pain control. The laser beam has effects for treating the wrinkle and ageing spots

### BRIEF DESCRIPTION OF THE DRAWINGS

Fig. 1 is an overall block diagram of an ultrasonic ion skin care apparatus of the first embodiment of the present invention.
Fig. 2 is a circuitry of power unit for the ultrasonic ion skin care apparatus of the first embodiment of the present invention.
Fig. 3 is a circuitry of display unit for the ultrasonic ion skin care apparatus of the first embodiment of the present invention.
Fig. 4 is a circuitry of ultrasonic generating unit for the ultrasonic ion skin care devise of the first embodiment of the present invention.
Fig. 5 is a circuitry of galvanic activator for the ultrasonic ion skin care apparatus of the first embodiment of the present invention.
Fig. 6 is a circuitry of power switching unit for the ultrasonic ion skin care apparatus of the first embodiment of the present invention.
Fig. 7 is a circuitry of sound signal generating unit for the ultrasonic ion skin treating apparatus of the first embodiment of the present invention.
Fig. 8 is the circuitries of ISP connecting unit and resetting unit for the ultrasonic ion skin treating apparatus of the first embodiment of the present invention.
Fig. 9 is an overall block diagram for the ultrasonic ion laser skin care apparatus of the second embodiment of the present invention.
Fig. 10 is a circuitry of power unit for the ultrasonic ion laser skin treating apparatus of the second embodiment of the present invention.
Fig. 11 is a circuitry of display unit for the ultrasonic ion laser skin treating apparatus of the second embodiment of the present invention.
Fig. 12 is a circuitry of laser beam generating unit for the ultrasonic ion laser skin care apparatus of the second embodiment of the present invention.
Fig.13 is a circuitry of low/high frequency pulse generating unit for the ultrasonic ion laser skin care apparatus of the second embodiment of the present invention.
Fig. 14 is a circuitry of ISP connecting unit and resetting unit for the ultrasonic ion laser skin care apparatus of the second embodiment of the present invention.
Fig. 15 is a schematic drawing to illustrate a single piece of ultrasonic vibrating probe, which has dual functions of ultrasonic diaphragm and ion contacting electrode, simultaneously of the present invention. Fig. 16 is illustrating an equivalent block diagram for the galvanic ion induction, the ultrasonic generation and the skin conductivity measurement of the skin treating apparatus of the present invention.
Fig. 17 is a flow chart for measuring the skin conductivity of the skin care apparatus of the present invention.

### THE DETAILED DESCRIPTION OFTHE PREFERRED EMBODIMENT

Hereinafter, an ultrasonic ion skin care apparatus referring to the first embodiment of the present invention will be described with the accompanying drawings 1 to 8.

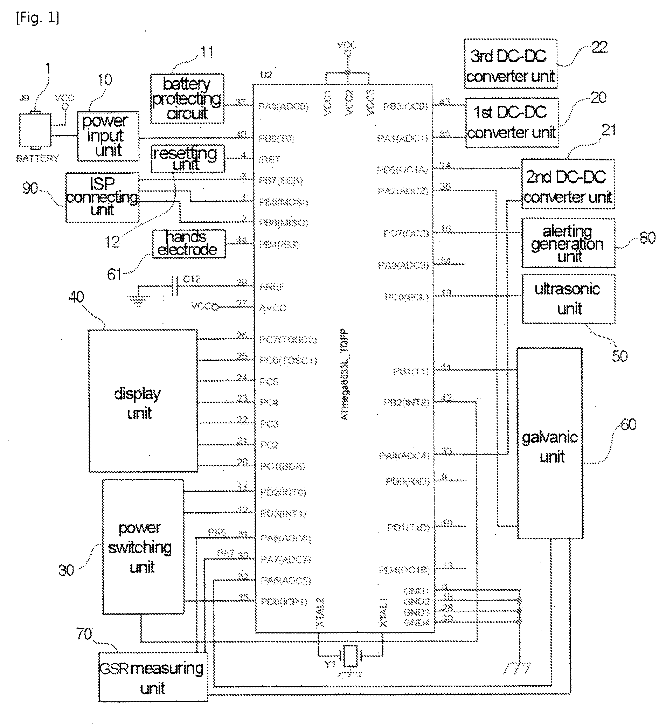

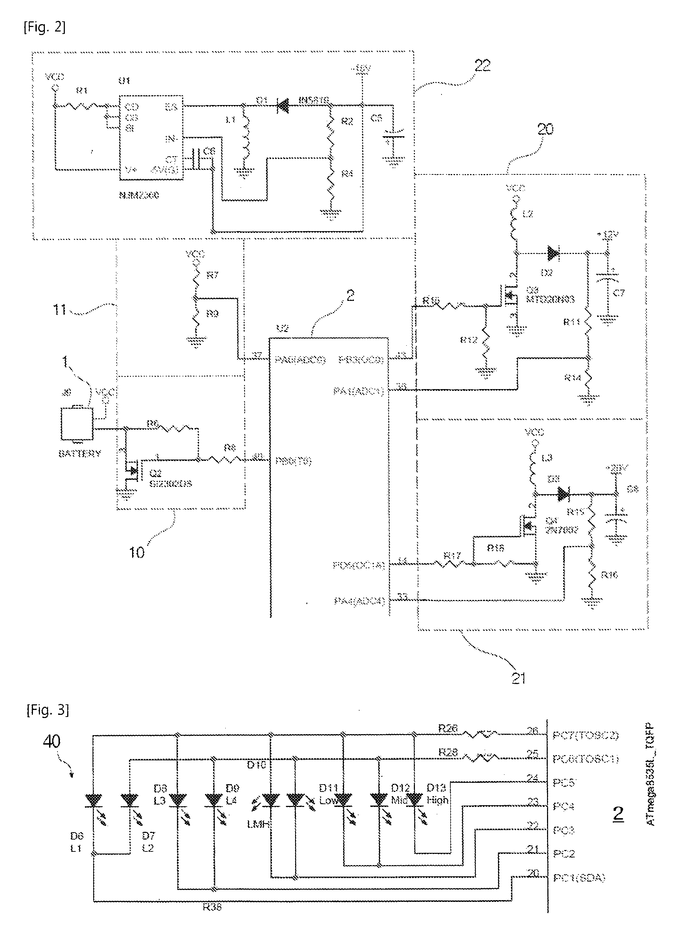

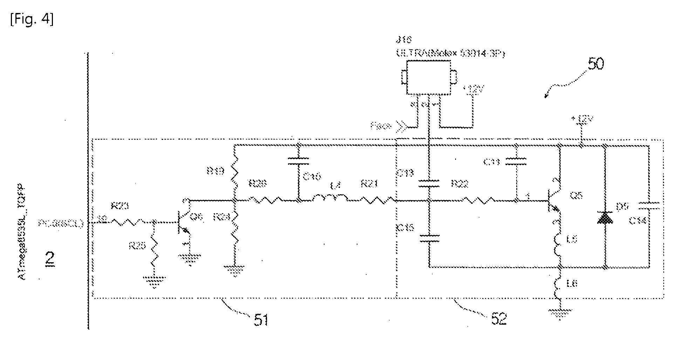

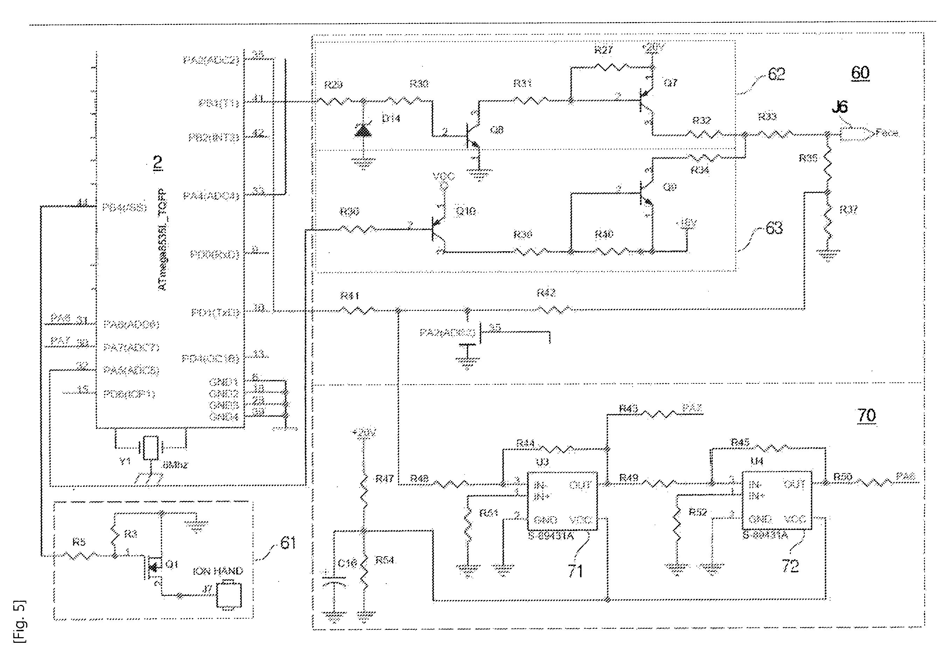

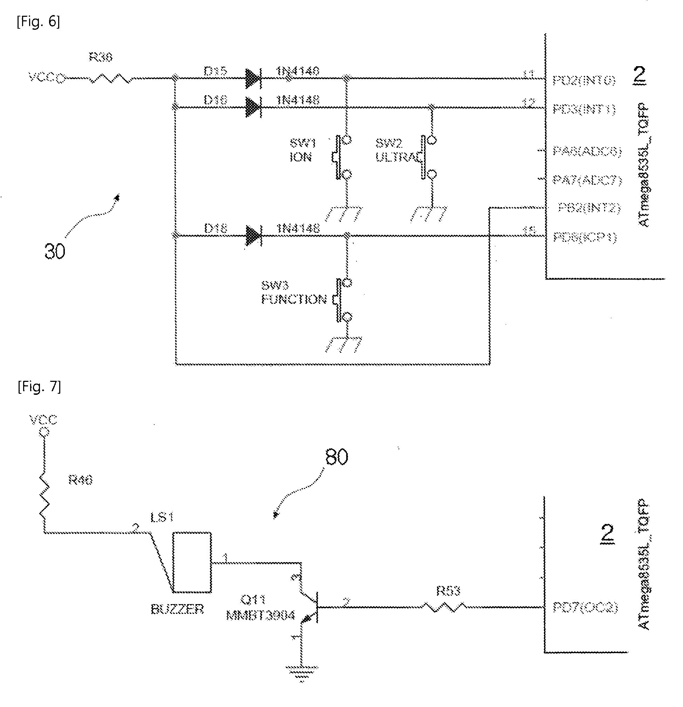

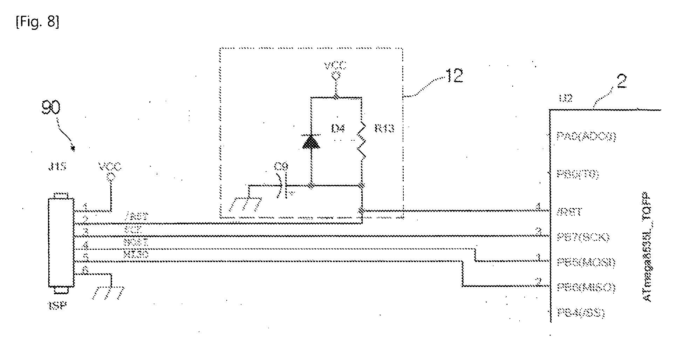

Referring to drawings, Fig. 1 is an overall block diagram of an ultrasonic ion skin care apparatus of the first embodiment of the present invention. Fig. 2 is a circuitry of power unit for the ultrasonic ion skin care apparatus. Fig. 3 is a circuit of display unit for the ultrasonic ion skin care apparatus. Fig. 3 is a circuit of display unit for the ultrasonic ion skin treating apparatus. Fig. 4 is a circuit of ultrasonic generating unit for the ultrasonic ion skin treating apparatus. Fig. 5 is a circuitry of galvanic activator for the ultrasonic ion skin treating apparatus. Fig. 6 is a circuitry of switching unit for the ultrasonic ion skin treating apparatus. Fig. 7 is a circuitry of sound signal generating unit for the ultrasonic ion skin care apparatus. Fig. 8 is the circuitries of ISP connecting unit and resetting unit for the ultrasonic ion skin treating apparatus of the first embodiment of the present invention.

First, as shown in Fig. 1, the battery (1) in the CPU (2) is connected to a power source (Vcc) through the power input unit (10) for activating the CPU (2). The CPU (2) controls the ultrasonic unit (50) for generating the ultrasonic wave; the galvanic unit (60) including the hand electrode (61), the galvanic power generating unit (62) and the waveform control circuit (63) for inducing the ion activated material into the skin by accessing galvanic ion current; the skin conductivity(GSR) measuring unit (70) for detecting the skin conductivity(GSR) by contacting the user's skin; the power switching unit (30) for power source and operating unit; the display unit (40) for displaying the operation status; the ISP connecting unit (90); the alerting generation unit (80) for sound warning; and the resetting unit (12) for re-booting the system.

On the other hand, the battery (1) of the CPU (2) is charged by connecting the power source (Vcc). The CPU (2) includes first and second DC-DC converter units (20, 21) and third DC-DC converter unit (22) having a separate circuit for generating various voltages to control the accurate operations and the battery protecting circuit (11) for detecting the power reserving and reducing the consumption of the power.

For example, the voltage (Vcc) of the battery (1) is boosted to +12V by first DC-DC converter unit (20) for operating the ultrasonic unit (50). The voltage (Vcc) is boosted to +20V by second DC-DC converter unit (21) for operating the galvanic unit (60) and the skin conductivity measuring unit (70). The voltage (Vcc) is boosted to -18V by the third DC-DC converter unit (22) for operating other units.

Referring to the Fig.2, a power unit, which is including the power input (10), the first-, second- and third DC-DC converter units (20, 21, 22) and the battery protecting circuit (11), will be described in detail.

An input terminal of power input unit (10) is connected in parallel to the terminal of the outside power source or the terminal of the battery (J9).

The power source (Vcc) of battery (1) is applied to the PBO terminal of CPU (2), through the switching element (Q2). The battery protecting circuit (11) comprises a battery voltage measuring circuit, which is consisted of the resistances (R7, R8). The battery protecting circuit (11) measures a battery voltage and transmits to an analog input terminal (PA0) of the CPU.

Meanwhile, the portable skin care equipment is commonly used a single input power (for example, 3.6V) because it adopts the battery power. In some cases (for instance, the ion induction uses 12-40V), it is essential to have a boosting process of the high voltage. The first- and second DC-DC converter units (20, 21) are controlled according to the switching elements (Q3, Q4) for adjusting the output voltage by adjusting the frequency duty rate and the PWM control program of the CPU, for outputting the 12V and 20V, respectively. (For example, second embodiment described later, 40V will output through the forth DC-DC converter unit (23), in the same way.)

In other words, when the switching signal issued from the PWM control terminal (OCO, OC1A terminal) of the CPU, the first- and second DC-DC converter units (20, 21) operate the switching element (D6), on and off, for controlling the pulse width, so that it is possible to control the output voltage.

In fact, the output voltages of DC-DC converters, it will output 12-40V, when the skin care apparatus is operating. But, it leaves power-on state without operating; it will maintain 10V for reducing the power consumption.

Meanwhile, the 3rd DC-DC converter unit (22) will boost the battery voltage (for example 3.6V) to -18V. The galvanic unit (60), which is described below, will stimulate the galvanic ions by pressurizing for absorbing the active substances into the skin.

However, the 3rd DC-DC converter unit (22) adopts the DC-DC converter chip (U1). The switching transistor (Q2) of voltage applying unit is driven by the standard voltage of the chip (U1). The peripheral circuit, around the chip includes other elements (the resistors R1, R2, R4, capacitors C5, C6, inductor L1, diode D1).

In addition, the partial resistances (R7, R9) detect the Vcc voltage for sensing the standard voltage to meet the CPU voltage (for example 3.3V).

For performing the above, the power management program of the CPU, is described in detail as of the applicant's prior Korean patent No. 507453. The disclosure of the prior patent may be referred to easily understand the further explanation of the present invention.

Referring to the Fig. 3, the display unit (40) of the first embodiment of the present invention is disclosed, which is displayed the ion pulse status of high level, middle level, and low level, when the ions induced to the user's skin.

Meanwhile, as shown in Fig. 5, the skin care apparatus of the present invention shows the various functions of the switching units (30), it is consisted of first switch (SW1) for inducing the galvanic ion, second switch (SW2) for generating the ultrasonic waves and third switch (SW3) for measuring the skin conductivity.

In Fig.7, reference number '80' means the alerting generation unit comprising a buzzer (LS1) and a driving circuit of the buzzer which will alarm the abnormal operating condition.

In Fig.8, reference number '80' means the ISP (In-System Programming) connection unit for programming the system and reference number '12' means a reset unit.

Now, as shown in Fig. 5, a galvanic unit (60) and a skin conductivity measuring unit (70), which are the major components of the present invention will be disclosed in detail.

First, the pulse wave form of the control signal from the output terminal (PB1) of the micro-controller (2) is amplified by amplifying circuits and the ultrasonic power generating unit (52) corresponding to the static voltage circuit (R29, D14), and then transmitted to the contacting electrodes (J6) through the resistors (R32 and R33) for applying to the skin (for example, face).

On the other hand, the pulse wave form of the control signal from the output terminal (PB4) of the micro-controller (2) is amplified by the amplifier circuit and then applied to the hand electrode (J7)

At this point, the PWM control signal from the input/output terminals (PA5) of the microcontroller is applied to the PWM control transistor (Q9, Q10) corresponding to the ultrasonic generating circuit (51). The collector side of the transistor (Q9, Q10) is connected to the skin electrode (J6). The output waveform will be formed, for example, the triangle wave, according to the PWM control signal.

In addition, if the signal of the terminal (PB4) is on 'high', the transistor (Q1) is 'on' state and the handle connecting electrode (J7) is on "ground" state. The skin connecting electrode (J6) generates the corresponding outputs. Eventually, the galvanic current is flown from hand to face for effectively inducing the ionic active substances to the skin.

In contrast, the signal of the terminal (PB4) is on 'low', the transistor (Q1) is 'off' state. The hand electrode (J7) blocks out the skin connecting the electrode (J6) for separating the low-frequency operation afterward.

In other words, when the galvanic ion is induced, a relatively low voltage, 9 ∼ 20V of the single short wave or the continuous wave of the low frequency wave is used as the concept of ion tophoresis. When the low frequency wave is used, the short wave of 50-100v is applied. The transistor (Q1) of the hand electrode is used for separating the high- and low- voltages when the event of the low frequency wave is generated.

That is, the single short voltage of the low-frequency wave is 50V or higher than that of a few tens of volts. If the low frequency wave is operated during the galvanic is used on the user's skin, the user is experienced the feeling of unpleasant tingling. To prevent such a problem, when the low frequency wave is used, the hand electrode is electrically separated.

As referring to Fig. 5, the skin conductivity detecting unit detects the current flowing from the skin contacting electrode (J6) to the hand electrode (J7) or vice versa, for measuring the skin conductivities (that is, ultimately, detects the degree of skin moisture). The signal detected from the skin electrode (J6) is amplified by amplifying circuits and first and second amplifiers (71, 72), then transmitted to the micro-controller (2) through the input terminals (PA7, PA6).

Referring to Fig. 4, the ultrasonic unit (50) is disclosed in detail. The ultrasonic unit is applied the control signal from the output terminal (PCO) of the microcontroller (2) to the switching transistor (Q6) of the ultrasonic generation circuit (51) through the resistance (R23) to operates the resonance transistor (Q5) of the ultrasonic power generating unit (52) with the ultrasonic actuator (ULTRA), the rest of element, the transistors (Q5, Q6), the ultrasonic activator and the peripheral circuit.

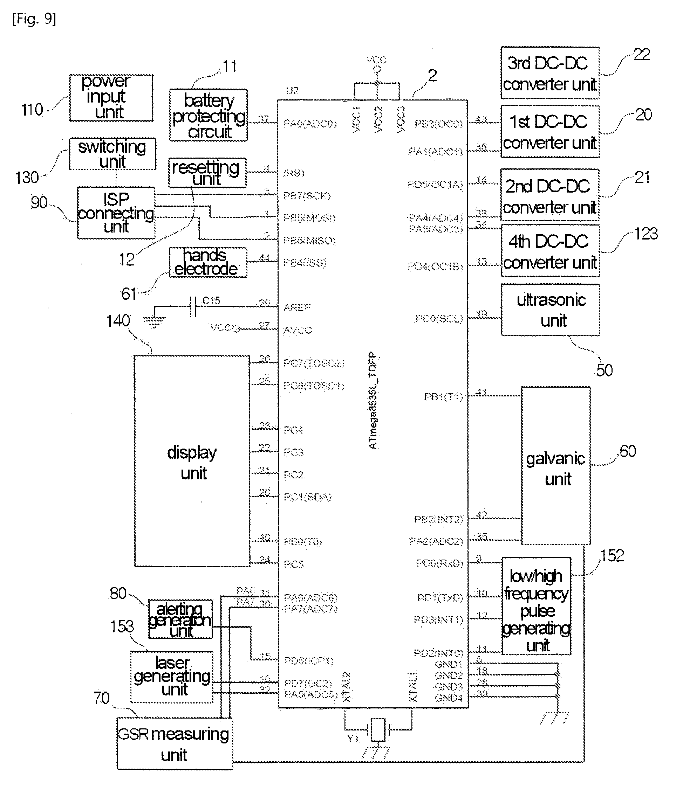

Hereinafter, the ultrasonic ion laser skin care apparatus of the second embodiment of the present invention is described in detail with referring to Fig. 9 to Fig. 14.

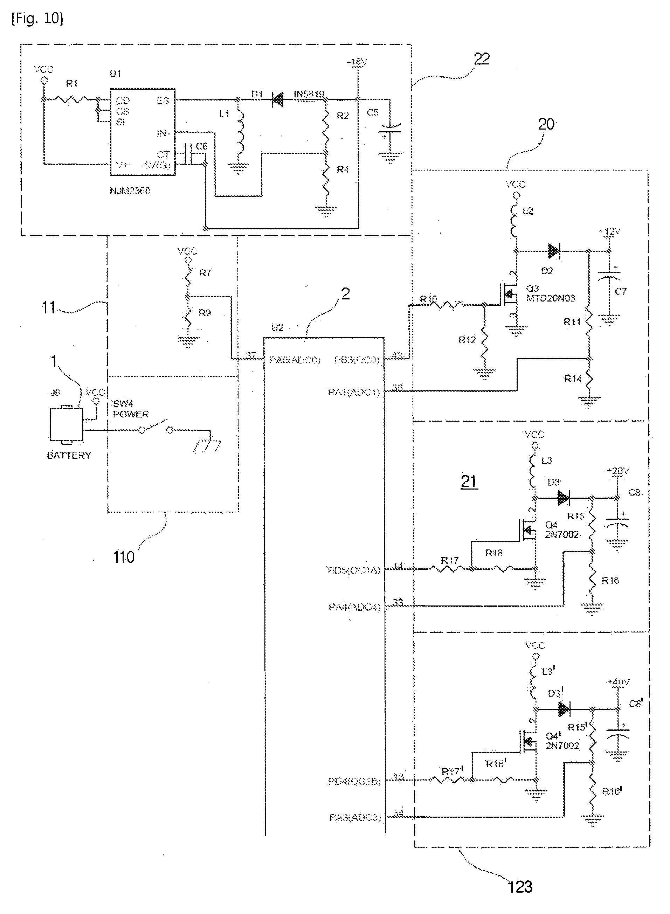

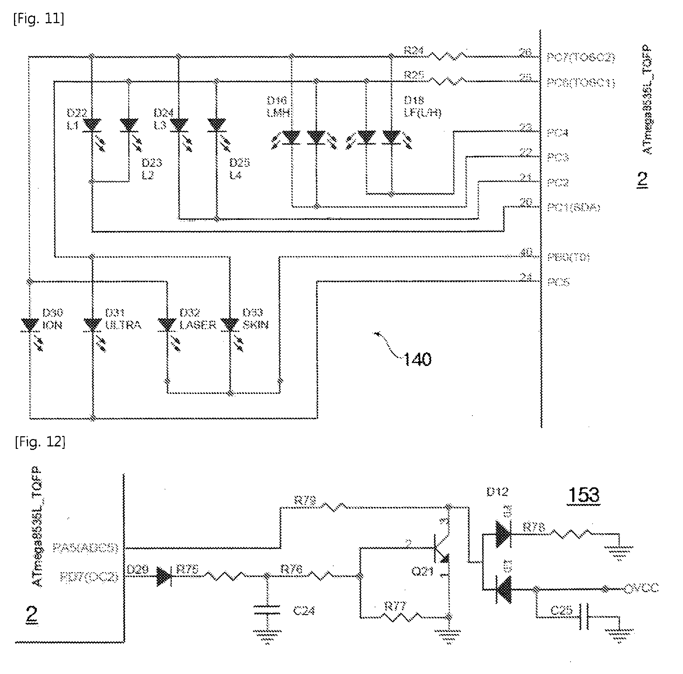

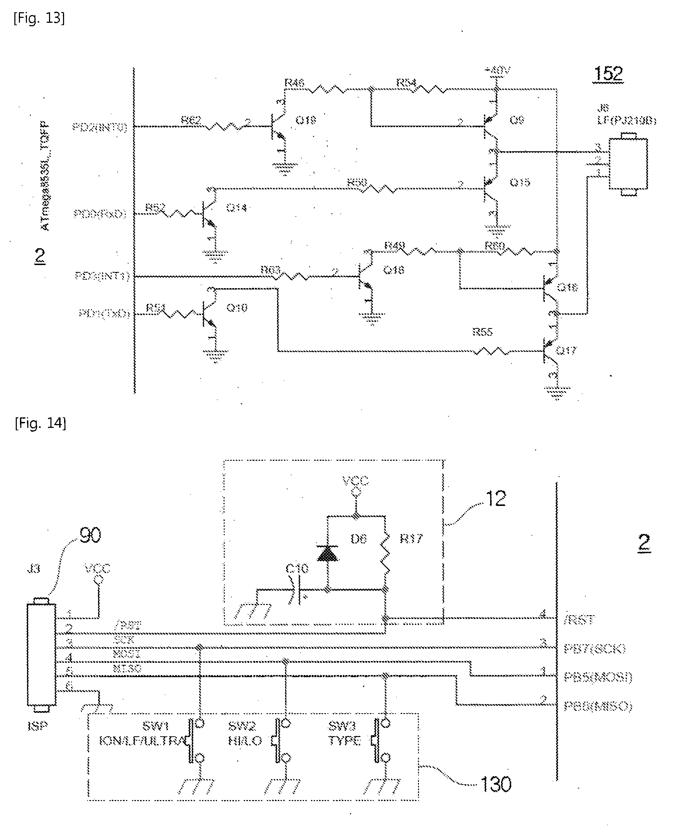

Fig. 9 is an overall block diagram for the ultrasonic ion laser skin care apparatus of the second embodiment of the present invention. Fig. 10 is a circuitry of the power unit for the ultrasonic ion laser skin treating care. Fig. 11 is a circuitry of display unit for the ultrasonic ion laser skin care. Fig. 12 is a circuitry of laser beam generating unit for the ultrasonic ion laser skin care. Fig. 13 is a circuitry of low/high frequency pulse generating unit for the ultrasonic ion laser skin care. Fig. 14 is a circuitry of the ISP connecting unit and resetting unit for the ultrasonic ion laser skin care apparatus of the second embodiment of the present invention.

For brief describing, the components of the second embodiment, which are similar to that of the first embodiment is omitted, but the differences are emphasized. The identical numeral references are used for the similar elements

As shown in Fig. 9, the ultrasonic ion laser skin care apparatus of the second embodiment of the present invention is added the features of the laser beam generator by comparing with the ultrasonic skin care apparatus of the first embodiment. Further, the low/high frequency pulse generating circuits are added. For those extra components, the forth DC-DC converter (123), the additional circuits and the connecting terminals of the CPU (2) are needed. Thus, there are slightly different in detail, each other.

First, as shown in Fig. 9 and Fig. 10, the power input unit (110) of the ultrasonic ion laser skin care devise of the second embodiment of the present invention is consisted of only the power switch (SW4), and it is only difference comparing with that of the first embodiment of the present invention. As mentioned before, when the switching signal is issued from the PWM control terminal (OC1B terminal) of the forth DC-DC converter (123), then the switching element (Q4 ') is operated, on/off for controlling the pulse width, so that it is possible to control the power output. When the skin care apparatus is operated, the output voltage is 40V, but the skin care apparatus is on without operation, the power maintains 10V for saving the power.

Referring to Fig. 11, the displaying unit (140) of the second embodiment is disclosed. In Fig. 11, the LED (D31, D30) for indicating the operation status of the ultrasonic unit (50) and the galvanic unit (60) for generating the galvanic ion, the LED (D16, D18) for displaying the high and low voltages, and the LED (D32, D33) for indicating the operation status of the laser generating unit and the low/high frequency pulse generating unit (153, 152).

Further, as shown in Fig. 14, the switching unit (130) and the ISP connecting unit (90) are disclosed. The switching unit (130) is consisted of: ion/ultrasonic switch (SW1), high/low switch (SW2) and the electrical conductivity operating switch (SW3). The galvanic unit (60) and the skin conductivity measuring unit (70) are identical configuration as that of the first embodiment.

Meanwhile, as shown in Fig. 12, the laser generating unit (153) is disclosed. A signal from the first laser output port (PA5) of the microcontroller (2) is applied to the photodiode (PD) and the laser diodes (LD) for controlling by the output of the second laser output port (PD7), and the switching transistor (Q21). The rest of them are the auxiliary elements

Then, the low/high frequency pulse generating unit (152) is applied the signal from the first low-/high frequency output terminal (PD2, PD0) of the microcontroller (2) to the low frequency resonance transistors (Q9, Q15). The signal from second low-/high frequency output terminal (PD3, PD1) of the microcontroller (2) is applied to a high frequency wave resonance transistor (Q16, Q17). A vibrational function is performed by applying the high frequency wave signal to the oscillating electrode (J6) according to the low frequency resonance transistor (Q16, Q18).

Preferably, it is efficient to operate the low/high frequency vibrating functions, at the same time when the skin care is in progress. It is also effective skin treatment to operate the laser function, together or individually.

On the hand, it is recommendable to have a single piece of the electrode, which simultaneously perform the ion inducing function and the ultrasonic vibrating function, together for efficient skin treatment as shown in the first embodiment and second embodiment

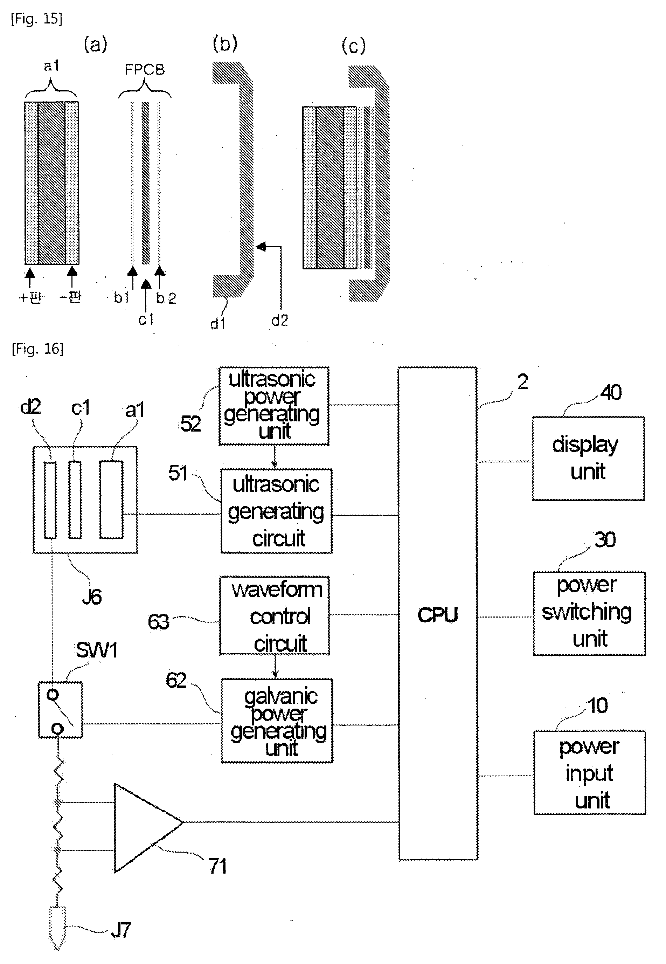

Fig. 15 shows a schematic drawing to illustrate a single piece of ultrasonic vibrating probe, which has dual functions of ultrasonic diaphragm and ion contacting electrode, simultaneously. Fig. 15 (a) show the laminate layers, which is disassembled status of the ultrasonic vibrating element, Fig. 15 (b) shows the cover for the ultrasonic vibrating element, and Fig. 15 (c) shows the assembly status of the ultrasonic vibrating element.

As mentioned before, it is possible to efficiently escalate the cosmetic effect by using the ultrasonic wave and the ions inducing, together. However, some conventional products have combined the ultrasonic function and ion inducting function, but it is operated the functions separately, each other. Those conventional products cannot operate the ultrasonic vibrating function and the ion inducing function, simultaneously.

Because the conventional skin care apparatus does not have the diaphragms, which is satisfied to fulfill these functions, at the same time. In order to fulfill the dual functions simultaneously, it may have a configuration and operational functions as shown in Fig. 15. That is, to have the ultrasonic vibrating function, the components are required that; an ultrasonic vibrating ceramic plate (a1) having one side of (+) plate and other side of (-) plate, the flexible PCB (FPCB) (b1, b2, c1), the ultrasonic vibration and ions transmitting head (d1), and the FPCB needed the SUS (c1) for insulating both sides of the micro-bond copper (b1, b2).

In addition, it is desirable that the diaphragm cover (d1) having surface (d2) being able to transmit the ultrasonic vibration, and having (+) pole for ion inducting function (when ion induced, the finger act as the (-) pole). It is desirable that the diaphragm cover (d1) is made of stainless steel, being able to transmit the ion current.

Considering the principle, it seems to possibly activate the function of the ultrasonic wave and ion inducting, simultaneously, if the both copper plate surfaces of the FPCB have the adhesive layer to form the insulating layer between the adhesive surfaces.

But, in reality, because the adhesive surfaces are in the dimension of the micrometer, the current is conducted. Therefore, it is required to use the materials, which have sufficient effect of the insulation. It is required to use the insulating materials, which is enough thin to be transmitted the ultrasonic wave energy (tens of micrometers) and have sufficient thermal resistance for high temperature of 120°. Such the materials, it is recommendable to use the polyimide film (65um) or polyester (70um).

Finally, as shown in Fig. 16, the operation of the galvanic ion inducting, the ultrasonic generating and the skin conductivity measuring of the present invention will be focused and described with reference to Figs. 4, 5 and 15, which are equivalent circuits. Fig. 16 shows an equivalent block diagram for the galvanic ion induction, the ultrasonic generation and the skin conductivity measurement of the skin care apparatus of the present invention.

As shown in Fig. 16, the ultrasonic wave operating signal of the ultrasonic generating circuit and the ultrasonic power generating unit (51, 52) is applied to the ultrasonic probe (a1) of the skin electrodes (J6). The galvanic current, which is applied through a switch (SW1) is flow through the ultrasonic probe (a1) and an insulator (c1), which are disposed between the conductors (d2) of the skin electrodes (J6), the galvanic power generating unit for inducing the galvanic ion and the waveform control circuit (62, 63) for inducing the galvanic ion.

On the other hand, the signal of the hands electrode unit (61) of a galvanic operating unit is applied to at least one of the hand electrode (J7). Then, it could be carried out the galvanic ion inducing and the ultrasonic wave vibrating on the user's skin, at the same time. At this point, the current between the hand electrode (J7) and the skin electrodes (J6) is detected by the resistances and amplified by the amplifier (71 and/or 72) of the electrical conductivity measuring unit to transmit the CPU (2). Therefore, it is possible to accurately anticipate the current level of user's skin moisture or the ion induction.

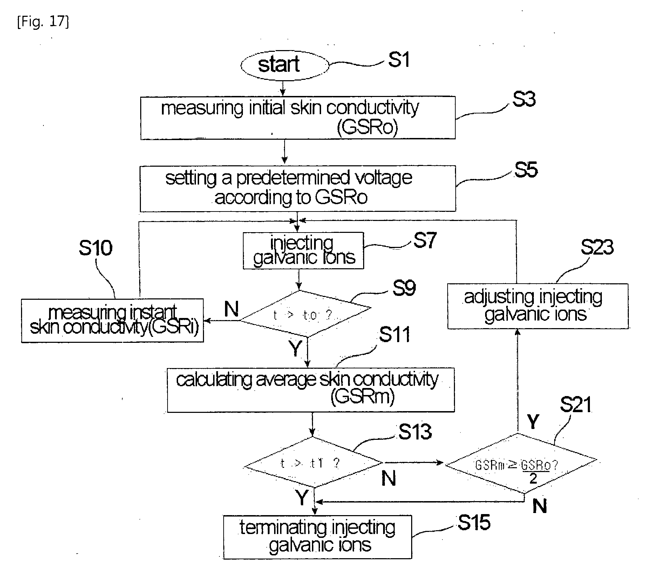

Additionally referring to Fig. 17, the operation of the skin care device, which has the measuring function of the skin conductivity and capable to carry out the ion induction of the present invention, will be described in the aspect of other point of view for utilizing them.

First, it starts the functions of the galvanic ion induction and the skin conductivity measurement (S1), then, measures the initial skin conductivity (GSRo) (S3).

Next, the skin will be categorized the status of "dry/semi-dry/oily" based on the user's initial skin conductivity for suitable treatment. Then, an appropriate ion inducing voltage is set according to the predetermined voltages(S5). Therefore, the initial ion inducing voltage is set 9~20V depending on the user's skin status of "dry/semi-dry/oily."

Next, according to the setting of the ion inducing voltage, the galvanic ion is injected to the user's skin (S7). Then, determine whether the ion inducing time is exceeded the predetermined time (to) (for example, 30 seconds) (S9). The ion is continuously injected within the predetermined time, at this moment, the skin conductivity (GSRi) is periodically measured within the constant interval, for example 10 times per a second (S10)

According to the above step (S9), the ion inducing time (to) (for example 30 seconds) is elapsed, the average value of the skin conductivity (GSRm) is calculated from the measured skin conductivity (S11), if the preset time for ion induction is passed, (t> t1) (S13), the ion injection terminates immediately (S15)

If not, the process goes back to step S21, and determine whether the above calculated average skin conductivity (GSRm) values is above the base value (e.g. half of initial skin conductivity (GSRo/2)). If the value is above the base value, the ion inducing voltage is adjusted to lower, for example by 1~2V (S23), then return back to repeating step S7~S13. If not, determine that the ion is fully induced on the skin, go to step S15, to terminate the ion injection.

Therefore, the skin care apparatus of the present invention is possible to automatically measure the user's skin conductivity to select the suitable ion inducing voltages and the ion inducing time for the user's skin conditions. It is also possible to check the intermediate skin conductivity during the skin treating operation. If the operator is determined that the ion is sufficiently induced into the user's skin, the operator may terminate the skin care operation, even if the preset time of the skin treatment is left.

However, when the preset time of the skin treatment (t1) is elapsed, the skin treating operation will be terminated forcibly, regardless of the skin conductivity. Therefore, it is desirable to set the sufficient preset time for the best skin treatment.

While the present invention has been described in connection with what is presently considered to be the most practical and preferred embodiment, it is to be understood that the present invention is not limited to the disclosed embodiment and the drawings, but, on the contrary, it is intended to cover various modifications and variations within the scope of the appended claims.

## Claims

1. An apparatus for skin care equipped with a measuring function of skin conductivity of a user (Galvanic Skin Response), the apparatus comprising:
at least one of DC-DC converter units (20, 21, 22, and 123) for boosting input voltage;
a galvanic unit (60) for applying a galvanic current between a hand electrode (J7) and a skin electrode (J6), said galvanic unit controlled and activated by the boosted voltage of said DC-DC converter units;
a switching unit (30, 130) for switching an operational mode of said galvanic unit; and
a microcontroller (2) for controlling the DC-DC converter units, the galvanic unit and the switching unit,
wherein, said apparatus for skin care further comprising a skin conductivity measuring unit (70) for measuring the user's skin conductivity by detecting currents from the contacted electrodes, and
**characterized in that** said microcontroller (2) is configured to: measure initial skin conductivity (GSRo); set a predetermined ion input voltage according to the measured initial skin conductivity; inject galvanic ions according to the predetermined ion input voltage; measure instant skin conductivity (GSRi) periodically with constant intervals, while the galvanic ions are being injected; calculate average skin conductivity (GSRm); adjust the injecting galvanic ions and reduce the ion input voltage, if a value of the calculated average skin conductivity (GSRm) is above a base value and if the injection time of the galvanic ions (t) has not exceeded the preset time (t1); and terminate the injecting galvanic ions, if a value of the calculated average skin conductivity (GSRm) is not above the base value or if the injection time(t) has exceeded the preset time (t1).

2. The apparatus for skin care, as claimed of claim 1, wherein the apparatus is further comprising an ultrasonic unit (50), which is controlled by the microcontroller.

3. The apparatus for skin care, as claimed of claim 1 or 2, wherein the apparatus is further comprising a laser generating unit (153), which is controlled by the microcontroller, for emitting a laser beam on the skin.

4. The apparatus for skin care, as claimed of claim 1 or 2, wherein the apparatus is further comprising a low/high frequency pulse generating unit (152) for generating electrical oscillations of the low or high frequency to stimulate the user's skin, said low/high frequency pulse generating unit (152) being controlled by the microcontroller,
wherein an electrical circuit (Q1) of the galvanic unit (60) will be shut down to disconnect the hand electrode (J7), when the low/high frequency pulse generating unit (152) operates.

5. The apparatus for skin care, as claimed of claim 2, wherein the apparatus is further comprising a skin contacting element for the ions deposition and the ultrasonic vibration (diaphragm and contacting electrode) made of a single piece, in order to accomplish the functions of the ions deposition and the ultrasonic vibration, simultaneously.

6. The apparatus for skin care, as claimed of claim 1, wherein the skin conductivity measuring unit (70) detects the current between the hand electrode (J7) and the skin electrode (J6) of the galvanic unit (60), amplifies the detected current by the amplifier (71, 72) and inputs the amplified current to the microcontroller (2).

7. The apparatus for skin care, as claimed of claim 1, wherein the base value is half of the initial skin conductivity (GSRo/2).

8. A method of operation for skin care equipped with a measuring function of skin conductivity of a user (Galvanic Skin Response), the method comprising the steps of:
(a) measuring initial skin conductivity (GSRo) (S3);
(b) setting a predetermined ion input voltage, which is suitable for skin to conduct the ions, according to the measured initial skin conductivity from the above step (a) (S5);
(c) injecting galvanic ions according to the predetermined ion input voltage setting from the above step (b) (S7);
(d) judging whether or not the injection time (t) of the galvanic ions is exceeded a preset time (t0) (S13);
(e) measuring instant skin conductivity (GSRi) periodically with constant intervals, while the galvanic ions are being injected (S10);
(f) calculating average skin conductivity (GSRm) according to the measured instant skin conductivities from the above step (e) (S11);
(h) judging whether or not a value of calculated average skin conductivity (GSRm) from the above step (f) is above a base value (S21); and
(i) adjusting the injecting galvanic ions and reducing the ion input voltage, if the value of calculated average skin conductivity (G8Rm) from the above step (h) is above the base value and if the injection time of the galvanic ions (t) is not exceeded the preset time (t1) (S23), then returning back to step (c) for repeating, if not, terminating; and
(j) terminating the injecting galvanic ions according to the above judging step (d) regardless of the value of calculated average skin conductivity (GSRm) from the above step (f), if it is determined the time elapsed the preset time (S15).

9. The method of operation for skin care, as claimed of claim 8, wherein the base value is half of the initial skin conductivity (GSRo/2).

## Patentansprüche

1. Ein Hautpflegegerät ausgestattet mit einer Funktion zum Messen der Leitfähigkeit von Haut eines Nutzers (galvanische Hautreaktion), das Hautpflegegerät aufweisend:
mindestens eine Gleichstrom-Gleichstrom Umwandlungseinheit (20, 21, 22 und 123) um die Eingangsspannung zu erhöhen;
eine galvanische Einheit (60) um galvanischen Strom zwischen einer Handelektrode (J7) und einer Hautelektrode (J6) einzuleiten, wobei die besagte galvanische Einheit mittels der erhöhten Spannung der besagten Gleichstrom-Gleichstrom Umwandlungseinheit gesteuert und aktiviert wird;
eine Schalteinheit (30, 130) um einen betriebsbereiten Modus der besagten galvanischen Einheit zu schalten; und
einen Mikrokontroller (2) um die Gleichstrom-Gleichstrom Umwandlungseinheit, die galvanische Einheit und die Schalteinheit zu steuern;
worin das genannte Hautpflegegerät weiter eine Hautleitfähigkeitsmesseinheit (70) aufweist um die Leitfähigkeit der Haut des Nutzers zu messen, indem Ströme von den kontaktierenden Elektroden ermittelt werden; und
**dadurch gekennzeichnet, dass** der genannte Mikrokontroller (2) dazu konfiguriert ist um: die Anfangsleitfähigkeit der Haut (GSRo) zu messen; eine festgelegte Ioneneingangsspannung aufgrund der gemessenen Anfangsleitfähigkeit festzulegen; galvanische Ionen gemäss der vorbestimmten Ioneneingangsspannung zu injizieren; eine gegenwärtige Hautleitfähigkeit (GSRi) periodisch in konstanten Intervallen zu messen, während die galvanischen Ionen injiziert werden; die durchschnittliche Hautleitfähigkeit (GSRm) zu berechnen; das Injizieren der Ionen einzustellen und die Ioneneingangsspannung zu reduzieren, falls ein Wert der berechneten Hautleitfähigkeit (GSRm) oberhalb eines Basiswerts liegt und falls die Injektionszeit (t) der galvanischen Ionen nicht über der vorgegebenen Zeit (t1) liegt; und das Injizieren der galvanischen Ionen zu stoppen, falls ein Wert der berechneten durchschnittlichen Hautleitfähigkeit (GSRm) nicht über dem Basiswert liegt oder falls die Injektionszeit (t) über der vorgegebenen Zeit (t1) liegt.

2. Das Hautpflegegerät gemäss Anspruch 1, worin das Gerät weiter eine Ultraschalleinheit (50) aufweist, die mittels des Mikrokontrollers gesteuert wird.

3. Das Hautpflegegerät gemäss Anspruch 1 oder 2, worin das Gerät weiter eine Laser generierende Einheit (153) aufweist, die mittels des Mikrokontrollers gesteuert wird, um einen Laserstrahl auf die Haut auszustrahlen.

4. Das Hautpflegegerät gemäss Anspruch 1 oder 2, worin das Gerät weiter eine tief/hoch Frequenz Impuls generierende Einheit (152) um elektrische Schwingungen der Hoch- oder Tieffrequenz zu generieren um die Haut des Nutzer zu stimulieren, aufweist, wobei die genannte tief/hoch Frequenz Impuls generierende Einheit (152) mittels des Mikrokontrollers gesteuert wird,
worin eine elektrische Schaltung (Q1) der galvanischen Einheit (60) abgeschaltet wird um die Handelektrode (J7) zu trennen, wenn die tief/hoch Frequenz Impuls generierende Einheit (152) läuft.

5. Das Hautpflegegerät gemäss Anspruch 2, worin das Gerät weiter ein Haut berührendes Element aus einem Stück gefertigt, für die Ionen Ablagerung und die Ultraschall Vibration (Diaphragma und kontaktierende Elektrode) aufweist, um die Funktionen der Ionen Ablagerung und der Ultraschall Vibration gleichzeitig auszuführen.

6. Das Hautpflegegerät gemäss Anspruch 1, worin die Hautleitfähigkeitsmesseinheit (70) den Strom der galvanischen Einheit (60) zwischen der Handelektrode (J7) und der Hautelektrode (J6) ermittelt, den ermittelten Strom mittels Verstärker (71, 72) verstärkt und den verstärkten Strom in den Mikrokontroller (2) einspeist.

7. Das Hautpflegegerät gemäss Anspruch 1, worin der Basiswert halb so gross ist wie die Anfangsleitfähigkeit der Haut (GSRo/2).

8. Eine Methode zum Betrieben von Hautpflege ausgerüstet mit einer Funktion zum Messen der Hautleitfähigkeit eines Nutzers (galvanische Hautreaktion), die Methode aufweisend die Schritte:
(a) Messen der Anfangshautleitfähigkeit (GSRo) (S3):
(b) Festlegen einer vorgegebenen Ioneneingangsspannung, die dazu geeignet ist damit die Haut Ionen leitet, gemäss der gemessenen Anfangshautleitfähigkeit des obigen Schritts (a) (S5);
(c) Injizieren von galvanischen Ionen gemäss der vorgegebenen, festgelegten Ioneneingangsspannung des obigen Schritts (b) (S7);
(d) Beurteilen ob die Injektionszeit (t) der galvanischen Ionen über der vorgegebenen Zeit (to) liegt (S13);
(e) Periodisches messen der gegenwärtigen Hautleitfähigkeit (GSRi) in konstanten Intervallen, während die galvanischen Ionen injiziert werden (S10);
(f) Berechnen der durchschnittlichen Hautleitfähigkeit (GSRm) gemäss der gemessenen gegenwärtigen Hautleitfähigkeiten des obigen Schritts (e) (S11);
(h) Beurteilen ob ein Wert der berechneten durchschnittlichen Hautleitfähigkeit (GSRm) des obigen Schritts (f) über einem Basiswert (S21) liegt; und
(i) Einstellen des Injizierens der galvanischen Ionen und reduzieren der Ioneneingangsspannung, falls der Wert der berechneten durchschnittlichen Hautleitfähigkeit (GSRm) des obigen Schritts (h) über dem Basiswert liegt und falls die Injektionszeit (t) der galvanischen Ionen nicht über der vorgegebenen Zeit (t1) (S23) liegt, dann zurückgehen zu Schritt (c) um zu repetieren, falls nicht, beenden; und
(j) Beenden des Injizierens von galvanischen Ionen gemäss des obigen Beurteilungsschritts (d) ohne Rücksicht auf den Wert der berechneten durchschnittlichen Hautleitfähigkeit (GSRm) des obigen Schritts (f) zu nehmen, falls festgestellt wird, dass die Zeit über der vorgegebenen Zeit liegt (S15).

9. Die Methode zum Betreiben von Hautpflege gemäss Anspruch 8, worin der Basiswert halb so gross ist wie die Anfangsleitfähigkeit (GSRo/2).

## Revendications

1. Un appareil pour le soin de la peau équipé avec une fonction de mesure d'une conductivité de la peau d'un utilisateur (réponse galvanique de la peau), l'appareil comprenant :
au moins une unité de conversion (20, 21, 22 et 123) courant continu - courant continu pour augmenter la tension d'entrée ;
une unité galvanique (60) pour appliquer un courant galvanique entre une électrode à main (J7) et une électrode de peau (J6), ladite unié galvanique est contrôlée et activée par la tension d'entrée augmentée de ladite unité de conversion courant continu - courant continu ;
une unité de commutation (30, 130) pour commuter un mode de fonctionnement de ladite unité galvanique ; et
un microcontrôleur (2) pour contrôler l'unité de conversion courant continu - courant continu, l'unité galvanique et l'unité de commutation ;
dans lequel ledit appareil pour le soin de la peau de plus comprend une unité de mesure la conductivité de peau (70) pour mesurer la conductivité de la peau d'un utilisateur en détectant les courants des électrodes contactées, et
**caractérisé en ce que** ledit microcontrôleur (2) est configuré pour : mesurer la conductivité de peau initiale (GSRo) ; régler la tension d'entrée prédéterminé des ions selon la conductivité de la peau initiale mésurée ; injécter des ions galvaniques selon la tension d'entrée des ions prédéterminé ; mesurer la conductivité de la peau instant (GSRi) périodiquement avec des intervalls constants, pendant que les ions galvaniques sont injectées ; calculer une conductivité de la peau moyenne (GSRm) ; ajuster l'injection des ions galvaniques et réduire la tension d'entrée des ions, si le valeur de la conductivité de la peau moyenne (GSRm) calculé est au-dessus d'un valeur de base et si le temps d'injection (t) des ions galvaniques n'a pas dépassé le temps prédéterminé (t1); et terminer l'injection des ions galvaniques, si le valeur de la conductivité de peau moyenne (GSRm) calculé n'est pas au-dessus le valeur de base ou si le temps d'injection (t) des ions galvaniques a dépassé le temps prédéterminé (t1).

2. L'appareil pour le soin de la peau selon la revendication 1, dans lequel l'appareil de plus comprend une unité ultrasonique (50), qui est contrôlée par le microcontrôleur.

3. L'appareil pour le soin de la peau selon la revendication 1 ou 2, dans lequel l'appareil de plus comprend une unité de généreration de laser (153), qui est contrôlée par le microcontrôleur, pour émettre un faisceau de laser sur la peau.

4. L'appareil pour le soin de la peau selon la revendication 1 ou 2, dans lequel l'appareil de plus comprend une unité de génération d'impulsions de haute/basse fréquence pour générer des oscillations électriques de la fréquence basse ou haute pour stimuler la peau d'un utilisateur, ladite unité de génération d'impulsions de haute/basse fréquence (152) étant contrôlée par le microcontrôleur,
dans lequel un cirucit électrique (Q1) de l'unité galvanique (60) sera fermé pour déconnecter l'électrode à main (J7), lorsque 1' unité de génération d'impulsions de haute/basse fréquence (152) fonctionne.

5. L'appareil pour le soin de la peau selon la revendication 2, dans lequel l'appareil de plus comprend un élement pour contacter la peau pour la déposition des ions et la vibration ultrasonique (diaphragme et électrode de contact) fabriqué en une seule pièce, afin de réaliser les fonctions de déposition des ions et de la vibration ultrasonique simultanément.

6. L'appareil pour le soin de la peau selon la revendication 1, dans lequel l'unité de mesure la conductivité de peau (70) détecte le courant entre l'électrode à main (J7) et l'électrode de peau (J6) de l'unité gavlanique (60), amplifie le courant détecté avec l'amplificateur (71, 72) et entre le courant amplifié dans le microcontrôleur (2).

7. L'appareil pour le soin de la peau selon la revendication 1, dans lequel la valeur de base est la moitié de la conductivité de peau initiale (GSRo/2).

8. Une méthode de fonctionnement pour le soin de la peau équipé avec une fonction de mesure d'une conductivité de la peau d'un utilisateur (réponse galvanique de la peau), la méthode comprenant les étapes de:
(a) mesurer d'une conductivité de peau initiale (GSRo) (S3) ;
(b) régler la tension d'entrée prédéterminé des ions, qui est adapté à la peau pour être conducteur des ions, selon la conductivité de peau initiale mesurée à partir de l'étape ci-dessus (a) (S5) ;
(c) injecter des ions galvaniques selon la mise de la tension d'entrée prédéterminé des ions à partir de l'étape ci-dessus (b) (S7) ;
(d) juger si le temps d'injection (t) des ions galvaniques a dépassé un temps prédéterminé (to) (S13) ;
(e) mesurer d'une conductivité de peau instant (GSRi), périodiquement avec des intervalls constants, pendant que les ions galvaniques sont injectées (S10) ;
(f) calculer d'une conductivité de peau moyenne (GSRm) selon les conductivités de peau instant (GSRi) mesurées à partir de l'étape ci-dessus (e) (S11) ;
(h)juger si un valeur de la conductivité de peau moyenne (GSRm) calculé à partir de l'étape au-dessus (f) est au-dessus un valeur de base (S21) ; et
(i) ajuster l'injection des ions galvaniques et réduire la tension d'entrée des ions, si le valeur de la conductivité de peau moyenne (GSRm) calculé à partir de l'étape au-dessus (h) est au-dessus le valeur de base et si le temps d'injection (t) des ions galvaniques n'est pas dépassé le temps prédéterminé (t1) (S23), ensuite retourner a l'étape (c) pour répéter, sinon, terminer ; et
(j) terminer l'injection des ions galvaniques selon l'étape de jugemement au-dessus (d) sans se soucier du valeur de la conductivité de peau moyenne (GSRm) calculée à partir de l'étape au-dessus (f), s'il est déterminé que le temps a écoulé le temps prédéterminé (S15).

9. La méthode de fonctionnement pour le soin de la peau selon la revendication 8, dans laquelle le valeur de base est la moitié de la conductivité de la peau initiale (GSRo/2).
